# EUROPEAN PATENT SPECIFICATION

(11) **EP 1 185 521 B1**
(45) Date of publication and mention of the grant of the patent: **06.10.2004**
(21) Application number: 01921931.0
(22) Date of filing: 20.04.2001
(51) Int. Cl.: C07D 277/18, C07D 277/40, C07D 401/06, C07D 417/06, C07D 213/73, C07D 233/88, C07D 403/06, A61K 31/426, A61K 31/427, A61K 31/505, A61K 31/506, A61K 31/4164, A61K 31/4178, A61K 31/44, A61K 31/4427, A61P 25/28

(54) **SUBSTITUTED 1-AZA-2-IMINO-HETEROCYCLES AND THEIR USE AS ACTIVATORS OF NICOTINIC ACETYLCHOLINE RECEPTORS**
SUBSTITUIERTE 1-AZA-2-IMINO-HETEROCYCLEN UND IHRE VERWENDUNG ALS AKTIVATOREN DER NIKOTINERGEN ACETYLCHOLINREZEPTOREN
1-AZA-2-IMINO-H T ROCYCLES SUBSTITU S ET LEUR UTILISATION COMME ACTIVATEURS DE R CEPTEURS NICOTINIQUES D'AC TYLCHOLINE

(30) Priority: 21.04.2000 JP 2000120975
(43) Date of publication of application: 13.03.2002
(73) Proprietor: Daiichi Suntory Pharma Co., Ltd., Tokyo 102-8530 (JP)
(72) Inventor: IMOTO, Masahiro, Tatebayashi-shi, Gunma 374-0029 (JP); IWANAMI, Tatsuya, Ashikaga-shi, Tochigi 326-0056 (JP); AKABANE, Minako, Ibaraki-shi, Osaka 567-0801 (JP); TANI, Yoshihiro, Ibaraki-shi, Osaka 567-0843 (JP)
(74) Representative: VOSSIUS & PARTNER
(86) International application number: PCT/JP2001/003377
(87) International publication number: WO 2001/081326

(56) References cited:
- EP-A- 0 679 397
- EP-A- 0 857 725
- WO-A-00/53582
- BE-A- 613 662
- US-A- 3 274 209
- US-A- 3 868 374
- US-A- 4 181 661
- US-A- 5 212 192
- MEAKINS G D ET AL: "Substituted imidazo[2,1-b]thiazoles from 2-aminothiazoles and alpha-bromo ketones: efficient preparation and proof of structure" JOURNAL OF THE CHEMICAL SOCIETY, PERKIN TRANSACTIONS 1, no. 3, March 1989 (1989-03), pages 643-648, XP002178318
- YALE H L ET AL: "1-Aralkyl-2(1H)pyridinimines and their derivatives" JOURNAL OF HETEROCYCLIC CHEMISTRY, vol. 12, no. 5, October 1975 (1975-10), pages 1027-1029, XP002178319
- JEN T ET AL: "Amidines. 4. Synthesis of tricyclic guanidines related to 1,2,3,5-tetrahydroimidazo(2,1-b) quinazoline, a new antihypertensive agent" JOURNAL OF MEDICINAL CHEMISTRY, vol. 16, no. 4, 1973, pages 407-411, XP002134186
- ANDREANI A ET AL: "6-Pyridinylimidazo[2,1-b]thiazoles and thiazolines as potential cardiotonic agents" EUROPEAN JOURNAL OF MEDICINAL CHEMISTRY - CHIMICA THERAPEUTICA, vol. 20, no. 1, 1985, pages 93-94, XP002178320
- CHEMICAL ABSTRACTS, vol. 61, no. 1, 6 July 1964 (1964-07-06) Columbus, Ohio, US; abstract no. 651h, WOLLWEBER H ET AL: "1-Phenethyl-2-iminoimidazolidines, a new class of compounds with ganglion-regulating activity" XP002178322 & MED. CHEM., ABHANDL. MED. CHEM. FORSCHUNGSSTAETTEN FARBWERKE HOECHST A.G., vol. 7, 1963, pages 248-261,
- D'AMOUR K A ET AL: "Desnitroimidacloprid and nicotine binding site in rat recombinant alpha-4 beta-2 neuronal nicotinic acetylcholine receptor" PESTICIDE BIOCHEMISTRY AND PHYSIOLOGY, vol. 64, no. 1, 1 May 1999 (1999-05-01), pages 55-61, XP002928879
- LATLI B ET AL: "Novel and potent 6-chloro-3-pyridinyl ligands for the alpha-4 beta-2 neuronal nicotinic acetylcholine receptor" JOURNAL OF MEDICINAL CHEMISTRY, vol. 42, no. 12, 17 June 1999 (1999-06-17), pages 2227-2234, XP002928877 cited in the application
- OKAZAWA A ET AL: "Prediction of the binding mode of imidacloprid and related compounds to house-fly head acetylcholine receptors using three-dimensional QSAR analysis" PESTICIDE SCIENCE, vol. 54, no. 2, October 1998 (1998-10), pages 134-144, XP002178321

## Description

### TECHNICAL FIELD

The present invention relates to compounds showing affinity for nicotinic acetylcholine receptors and activating the same. The compounds of the present invention are useful for preventing or treating of neurodegenerative diseases such as Alzheimer's disease and Parkinson's disease, dementia such as cerebrovascular dementia, motor ataxia such as Tourette's syndrome, neurosis during chronic cerebral infarction stage, neuropathy and mental disorder such as anxiety and schizophrenia and cerebral dysfunction caused by cerebral injury.

### BACKGROUND ART

It has been widely known that nicotine exerts a wide variety of pharmacological effects. These include, for example, cholinergic nervous activation as the effect on central nervous systems such as facilitation of acetylcholine release [De sarno P. & Giacobini E., *J. Neurosci. Res.,* 22, 194-200 (1984)], and further, activation effect on monoaminergic nervous systems [Levin E. D. & Simon B. B., *Psychopharmacology*, 138, 217-230 (1998)].

It has been also reported that nicotine possesses lots of very useful cerebral function improving effects such as increasing cerebral blood flow and glucose uptake rate in brain [Decker M. W. et al., *Life Sci*., 56, 545-570 (1995)].

It has been further reported that nicotine inhibits amyloid formation of β-peptides which is believed to be the cause of neuronal cell death during Alzheimer's disease [Salomon A. R. et al., *Biochemistry*, 35, 13568-13578 (1996)], and have cell protective effects on neuronal cell death induced by β-amyloid (Aβ) [Kihara T. et al., *Ann. Neurol*., 42, 156-163 (1997)]. Recent studies suggest the possibility of nicotine being a remedy for the inflammatory colitis [Sandborn W. J. et al., *Ann. Intern. Med*., 126, 364 (1997)].

On the other hand, it is acknowledged that in the patients of Alzheimer's disease, the degeneration of acetylcholinergic neurons known to be one of the important nervous systems responsible for cognition such as attention, learning, memory and recognition, is altered and thus nicotinic acetylcholine receptors in the cerebral cortex and hippocampus are drastically decreased [Nordberg A. et al., *J. Neurosci*. *Res.,* 31, 103-111 (1992)].

It is reported the possibility of the useful treatment for Alzheimer's disease by activating nicotinic acetylcholine receptors to be recovered the acetylcholine nervous systems mechanism by agonists or modulators of nicotinic acetylcholine receptors [Newhouse P. A. et al., *Psychopharmacology*, 95, 171-175 (1988)].

The nicotinic acetylcholine receptors belong to the ion channel neurotransmitter receptors composed of five subunits. That is, agonists such as acetylcholine, nicotine and the like are bound to receptors to activate and open the channels thereof, thus causing the influx of cationic ion such as sodium ion from extracellular to result the cell excitation [Galzi J. L. & Changeux J. P., *Neuropharmacology*, 34, 563-582 (1995)]. The aforementioned agonists such as acetylcholine, nicotine and the like show its effect by binding to the specific site existing in α subunit so-called agonist binding site.

It is known, on the other hand, that compounds such as galantamine and so on which activate cells by potentiating the effects of acetylcholine, have no agonist effect at nicotinic acetylcholine receptors directly. These compounds show their effects through allosteric site which is clearly different from the agonist binding sites [Schrattenholz A. et al., *Mol. Pharmacol*., 49, 1-6 (1996)].

Mentioned above, compounds capable to activate nicotinic acetylcholine receptors indirectly are called modulators and it is expected to be the practical medicines for treatment of the various neurological diseases [Lin N. -H & Meyer M. D., *Exp. Opin. Thr. Patents,* 8, 991-1015 (1998)].

The terms "agonists" and "modulators" are used in these definitions in the present specification.

The nicotinic acetylcholine receptors are believed to participate not only in Alzheimer's disease, but also in neurodegenerative diseases such as Parkinson's disease, and many of the neuroses and psychoses such as dementia, anxiety, schizophrenia and so on [Barrantes F. J., *in The Nicotic Acetylcholine Receptor*, ed. Barrantes F. J., Springer, 1997, p175-212; Lena C. & Changeux J. -P., *J. Physiol. (Paris)*, 92, 63-74 (1998)].

Especially, since it is known that cerebral blood flow of the patients suffering from cerebrovascular dementia caused by cerebral infarction is decreased [Takagi Shigeharu, *Gendai Iryo*, 28, 1157-1160 (1996); Tachibana H. et al., *J. Gerontol*., 39, 415-423 (1984)], there seems to be the possibility of agonists of nicotinic acetylcholine receptors or the modulators possessing cerebral blood flow increasing effect to be applied to the medicines in this area of treatment. Furthermore, recent study revealed that agonists of nicotinic acetylcholine receptors and the modulators thereof show analgesic activities [Bannon A. W. et al., *Science*, 279, 77-81 (1998)].

Nicotine itself surely affects as the agonist of nicotinic acetylcholine receptors. For example, after administration of nicotine to the patients of Alzheimer's disease, the recoveries of their attention or the short-term memory were observed, and also the symptoms of their disease were improved [Newhouse P. A. et al., *Drugs & Aging,* 11, 206-228 (1997)]. Nevertheless, nicotine also possesses disadvantages such as widely recognized addiction, as well as low bioavailability and severe side effects to the cardiovascular systems.

Therefore, there have been great expectation to develop nicotinic acetylcholine receptors agonists or modulators as medicines in place of nicotine which has no addiction, high bioavailability, and less side effects on cardiovascular systems [Maelicke A. & Albuquerque E. X., *Drug Discovery Today*, 1, 53-59 (1996); Holladay M. W. et al., *J. Med. Chem*., 40, 4169-4194 (1997)].

There are some subtypes known as the nicotinic acetylcholine receptors [Shacka J. J. & Robinson S. E. T., *Med. Chem. Res.,* 1996, 444-464], and mainly α4β2 subtype receptors exist in central nervous systems. Furthermore, there exist α1β1γδ (or α1β1εδ) subtype receptors in the neuromuscular junction of motor neurons, and α3β4 subtype receptors in ganglion of autonomic nervous systems and adrenal.

The activation of the cholinergic nervous systems and increasing effect of cerebral blood flow are believed to occur though α4β2 subtype receptors in central nervous systems, and above mentioned effects of nicotine on cardiovascular system are induced by affecting receptor subtypes exist in peripheral nervous system.

Therefore, it may be extremely useful as medicines having no side effects to develop compounds which have no affinity at α1β1γδ subtype nor α3β4 subtype receptors, but selectively affects α4β2 subtype receptors.

In these circumstances, there have been many proposals to develop selective agonists or modulators at nicotinic acetylcholine receptors of central nervous system as practical medicines. These include, for example, the compound such as ABT-418 [Arneric S. P. et al., *J. Pharmacol. Exp, Ther*., 270, 310-318 (1994); Decker M. W. et al., *J. Pharmacol. Exp. Ther*., 270, 319-328 (1994)], ABT-089 [Sullivan J. P. et al., *J. Pharmacol. Exp. Ther*., 283, 235-246 (1997); Decker M. W. et al., *J. Pharmacol. Exp. Ther*., 283, 247-258 (1997)], GTS-21 [Arendash G. W. et al., *Brain Res.,* 674, 252-259 (1995); Briggs C. A. et al., *Pharmacol. Biochem. Behav.,* 57, 231-241 (1997)], RJR-2403 [Bencherif M. et al., *J. Pharmacol. Exp. Ther*., 279, 1413-1421 (1996); Lippiello P. M. et al., *J. Pharmacol. Exp. Ther*., 279, 1422-1429 (1996)], SIB-1508Y [Cosford N. D. P. et al., *J. Med. Chem*., 39, 3235-3237 (1996); Lloyd. G. K. et al., *Life Sci*., 62, 1601-1606 (1995)], SIB-1553A [Lloyd. G. K. et al., *Life Sci*., 62, 1601-1606 (1995)] and so on.

In European Patent Publication EP679397-A2, substituted amine derivatives represented by the following formula were proposed for the medicines for prevention and treatment of cerebral dysfunction. 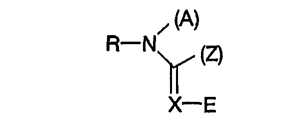 in which,
- R: represents hydrogen, optionally substituted acyl, alkyl, aryl, aralkyl, heteroaryl or heteroarylalkyl radicals;
- A: represents a monofunctional group of the hydrogen, acyl, alkyl or aryl series or represents a bifunctional group which is linked to the radical Z;
- E: represents an electron-withdrawing radical;
- X: represents the -CH= or =N- radicals, it being possible for the -CH= radical to be linked to the Z radical instead of an H atom;
- Z: represents a monofunctional group of the alkyl, -O-R, -S- R or -NR₂ series or represents a bifunctional group which is linked to the A radical or the X radical.

However, in the compounds disclosed in said patent publication, the E radical is restricted to the electron-withdrawing radical, and therefore, these compounds are clearly different from the compounds disclosed by the present patent application. Furthermore, there is no description in the above-mentioned patent publication that these compounds can selectively activate α4β2 nicotinic acetylcholine receptors.

On the other hand, "imidacloprid", as a pesticide, is known to have the similar skeleton as the compounds of the present invention. It is confirmed that the imidacloprid electrophysiologically affects as partial agonist at nicotinic acetylcholine receptors of PC12 cell [Nagata K. et al., *J*. *Pharmacol. Exp. Ther*., 285, 731-738 (1998)], and imidacloprid itself or its metabolites and their analogues possess affinity to the nicotinic acetylcholine receptors in mouse brain [Lee Chao S. & Casida E., *Pestic. Biochem. Physiol*., 58, 77-88 (1997); Tomizawa T. & Casida J. E., *J. Pharmacol*., 127, 115-122 (1999); Latli B. et al., *J. Med. Chem.,* 42, 2227-2234 (1999)], however, there is no report of the imidacloprid derivatives selectively activating α4β2 nicotinic acetylcholine receptors.

Furthermore, among the nitromethylene type pesticides, the following compounds were synthesized to optimize the carbon chain length (n=0 to 3) between 6-chloro-3-pyridyl group and nitromethylene imidazoline group, and it was reported that insecticidal activities of the compound in which n is 2 was weaker than the compound in which n is 1 (Nishimura K. et al., *Pestic. Biochem. Physiol*., 50, 51-59 (1994)).

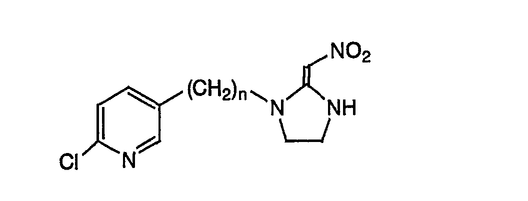

Japanese Laid-open Patent Publication Number Hei 10-226684 disclosed [N-(pyridinylmethyl)heterocyclic]ylideneamine compounds represented by the following formula, pharmaceutically acceptable salts and prodrugs thereof. 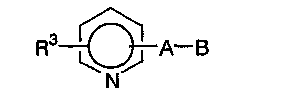 in which,
- A: represents the -CH(R)-;
- R³: represents a hydrogen atom or an optionally substituted C₁-C₆ alkyl; and
- B: represents the group of the following formula:

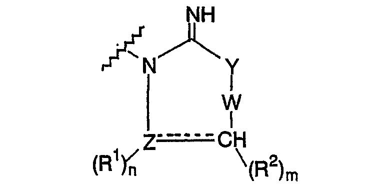

Nevertheless, among the compounds disclosed in said patent publication, the radical "A" which connects substituted pyridine ring and the radical "B", is substituted methylene group represented by -CH(R)-, and therefore, these compounds are clearly different from the compounds of the present invention in which the linkage is composed of substituted ethylene bridge. It is disclosed that aforementioned compounds possess weak affinity to nicotinic receptors; however, there is no disclosure that these compounds have selective activating effect at α4β2 nicotinic acetylcholine receptors of central nervous systems and act as agonists or modulators of nicotinic acetylcholine receptors.

Meakins et al, J. Chem. Soc. Perkin. Trans 1, 1989, pages 643-648 relate to the efficient preparation and proof of structure of substituted imidazo [2,1-b] thiazoles from 2-aminothiazoles and α-bromo ketones.

USA 3,274,209 discloses 6-substituted-imidazo [2,1-b] thiazole compounds.

USA 5,212,192 discloses immunostimulating 6-aryl-5,6-dihydroimidazo [2,1-b] thiazole derivatives.

Yale et al, Journal of Heterocyclic Chemistry vol. 12, No. 5, 1975, p. 1027-1029 pertain to 1-aralkyl-2(1H)pyrimidins and their derivatives.

Jen et al, Journal of Medical Chemistry, vol. 16, No. 4, 1973, pages 407-411 studied the influence of structural modification of 1, 2, 3, 5-tetra hydroimidazo [2,1-b] quinazoline on the antihypertensive activity.

Eur. J. Med. Chem., 1958 vol. 20, No. 1, pages 93-94 by Andreani and Ranbaldi discloses 6-pyridinylimidazo [2,1-b]thiazoles and thiazolines as potential cardiotonic agents.

Wollweber et al, Chemical Abstracts vol. 61, No. 1, 6. July 1964, Columbus Ohio; abstract number 651 h pertains to 1-phenethyl-2-iminoimidazolidines, a new class of compounds with ganglion-regulating activity.

USA 4,181,661 discloses derivatives of 2-iminothiazolindines and thin azolines.

BE 613 662 relates to a process for preparation of 2-imino-1,3-diaza-cycloalkanes.

USA 3,868,374 discloses new components having a central nervous (CNS) stimulating activity.

As mentioned above, there had been many attempts to develop agonists or modulators selectively activating α4β2 nicotinic acetylcholine receptors of central nervous systems via oral administration, but none were satisfactory.

### DISCLOSURE OF THE INVENTION

Therefore, the present invention provides therapeutic or preventing agents for treatment of diseases which may be prevented or cured by activating nicotinic acetylcholine receptors, having the capabilities of binding selectively with α4β2 nicotinic acetylcholine receptor of central nervous systems, and having no undesirable side effects in cardiovascular systems such as hypertension or tachycardia.

More specifically, the present invention provides medicaments for preventing or treating various diseases, which may be prevented or cured by activating nicotinic acetylcholine receptors, such as dementia, senile dementia, presenile dementia, Alzheimer's disease, Parkinson's disease, cerebrovascular dementia, AIDS-related dementia, dementia in Down's syndrome, Tourette's syndrome, neurosis during chronic cerebral infarction stage, cerebral dysfunction caused by cerebral injury, anxiety, schizophrenia, depression, Huntington's disease, pain and so on.

Through extensive investigations of researching compounds having the capabilities of binding selectively with α4β2 nicotinic acetylcholine receptors of central nervous systems, the present inventors discovered that the compounds represented by the formula (I) mentioned below and pharmaceutically acceptable salts thereof possess high affinity for nicotinic acetylcholine receptors in central nervous systems, and activate said receptors as agonists or modulators.

Accordingly, as one aspect of the present invention, it is provided heterocyclic compounds represented by the following formula (I): 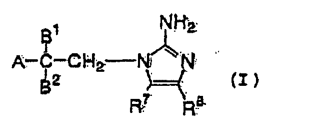 wherein:
A is an alkyl group selected from methyl, ethyl, propyl, isopropyl, butyl, sec-butyl and tert-butyl; an aryl group which may be substituted by a substituent selected from halogen atom, C₁-C₄ alkyl group, hydroxy group, alkylthiogroup and arylthiogroup; a 5-6 membered heterocyclic group or a condensed heterocyclic group thereof containing the same or different one to three heteroatom(s) of sulfur, nitrogen or oxygen atom(s), which may be substituted by C₁-C₄ lower alkyl group or halogen atom;
B¹ and B² are, hydrogen atom; alkyl group; or hydroxyl group; or combined together to represent carbonyl group;
the group -Y-X- is -C(R⁷)=C(R⁸)-N=, R⁷, R⁸ are hydrogen atom;
halogen atom; alkyl group selected from methyl, ethyl, propyl, isopropyl, butyl, sec-butyl and tert-butyl; aryl group which is either a non-substituted phenyl group or a phenyl group which is substituted by halogen atom or C₁-C₄ alkyl group; or 5- or 6- membered heterocyclic group); containing the same or different one to three heteroatom(s) of sulfur, nitrogen or oxygen atom(s), which may be substituted by C₁-C₄ lower alkyl group or halogen atom; or pharmaceutically acceptable salts thereof.

Still another aspect of the present invention, it is provided activator agents for α4β2 nicotinic acetylcholine receptors containing heterocyclic compounds of the formula (I) or pharmaceutically acceptable salt thereof as active ingredients.

As still further aspect of the present invention, it is provided that the use of heterocyclic compounds of the formula (I) or pharmaceutically acceptable salt thereof for treating or preventing of cerebral circulation disease, neurodegenerative disease and the like.

### BEST MODE FOR CARRYING OUT THE INVENTION

Examples of the pharmaceutically acceptable salt include an inorganic acid salt such as hydrochloric acid salt, hydrobromic acid salt, sulfuric acid salt, phosphoric acid salt and the like, and an organic acid salt such as fumaric acid salt, maleic acid salt, oxalic acid salt, citric acid salt, tartaric acid salt, malic acid salt, lactic acid salt, succinic acid salt, benzoic acid salt, methanesulfonic acid salt, p-toluenesulfonic acid salt and the like.

The group represented by "A" in the compound of formula (I) is an alkyl group, optionally substituted aryl group or heterocyclic group, wherein said alkyl group is selected from methyl, ethyl, propyl, isopropyl, butyl, sec-butyl and tert-butyl.

The preferable examples of aryl group may include phenyl, naphthyl and the like. Substituents of substituted aryl is selected from C₁-C₄ lower alkyl, hydroxyl group, alkylthio group, arylthio group, halogen atom , and therefore, examples of said substituted aryl group include methylphenyl, hydroxyphenyl, (methoxyphenyl)thiophenyl, (hydroxyphenyl)thiophenyl, chlorophenyl, dichlorophenyl and the like.

The term "heterocyclic group" represented by "A" is a 5 or 6 membered heterocyclic group or condensed heterocyclic group thereof containing the same or different 1 to 3 hetero atom(s) such as sulfur, nitrogen, oxygen atom(s), and examples include thiophene, furan, pyran, pyrrole, pyrazole, pyridine, pyrimidine, pyrazine, pyridazine, imidazole, oxazole, isoxazole, thiazole, isothiazole, quinoline, isoquinoline, indole, azaindole, tetrahydropyrimidine and the like.

Substituents of above heterocyclic group include C₁-C₄ lower alkyl, halogen atom , and therefore, examples of said substituted heterocyclic group may be 2-methylpyridine, 3-methylpyridine, 2-chloropyridine, 2-fluoropyridine, 2-bromopyridine, 3-bromopyridine, 2,3-dichloropyridine, 4-chloropyrimidine, 2-chlorothiazole, 3-methylisoxazole and the like.

The groups represented by "B¹" and "B²" in the compound of formula (I) are hydrogen atom, alkyl group or hydroxyl group; or combined together to represent carbonyl group. Preferable examples of alkyl group include C₁-C₄ lower alkyl such as methyl, ethyl, propyl, isopropyl, butyl, sec-butyl and the like.

The term "-Y-X-" is -C(R⁷)=C(R⁸)-N= (in which, R⁷, R⁸, are hydrogen atom; halogen atom; alkyl group selected from methyl, ethyl, propyl, isopropyl, butyl, sec-butyl and tert-butyl; aryl group which is either a non-substituted phenyl group or a phenyl group which is substituted by halogen atom or C₁-C₄-alkyl group; or 5- or 6-membered heterocyclic group containing the same or different one to three heteroatom(s) of sulfur, nitrogen or oxygen atom(s), which may be substituted by C₁-C₄ lower alkyl group or halogen atom.

The term "halogen atom" represented by R⁶, R⁷, R⁸, may include fluorine, chlorine, bromine and iodine.

The following are examples of heterocyclic compounds of the formula (I).
2-amino-1-[2-(6-chloro-3-pyridyl)ethyl]imidazole;
2-amino-1-[2-(6-methyl-3-pyridyl)ethyl]imidazole;
2-amino-1-[2-(5,6-dichloro-3-pyridyl)ethyl]imidazole;
2-amino-1-[2-(3-pyridyl)ethyl]imidazole;
2-amino-1-[2-(4-chlorophenyl)ethyl]imidazole;
2-amino-1-[2-(2-pyridyl)ethyl]imidazole;
2-amino-1-[2-(4-pyridyl)ethyl]imidazole;
2-amino-1-[2-(6-chloro-3-pyridyl)ethyl]-4,5-dimethylimidazole;
2-amino-1-[2-(2-chloro-5-thiazolyl)ethyl]imidazole;
2-amino-1-[2-(5-bromo-3-pyridyl)ethyl]imidazole;
2-amino-1-[2-(4-hydroxyphenyl)ethyl]imidazole;
2-amino-1-[2-(5-methyl-3-pyridyl)ethyl]imidazole;
2-amino-1-[2-(5-pyrimidyl)ethyl]imidazole;
2-amino-1-[2-(3-pyridazinyl)ethyl]imidazole;
2-amino-1-[2-(2-pyrazinyl)ethyl]imidazole;
2-amino-1-{2-[2-(4-hydroxyphenyl)thiophenyl]ethyl}imidazole;
2-amino-1-{2-[2-(4-methoxyphenyl)thiophenyl]ethyl}imidazole;
2-amino-1-[2-(4-pyridazinyl)ethyl]imidazole;
2-amino-1-[2-(4-chloro-5-pyrimidyl)ethyl]imidazole.

The heterocyclic compounds represented by the formula (I) of the present invention can be prepared in accordance with the various synthetic processes such as following Process 1 to 3.

In the following reaction schemes, the groups A, B¹, B², X, Y and n have the same meanings mentioned above.

### Process 1:

In accordance with the following reaction scheme, the compound of the formula (II) is reacted with the compound of the formula (III) to obtain the compound (I) of the present invention. 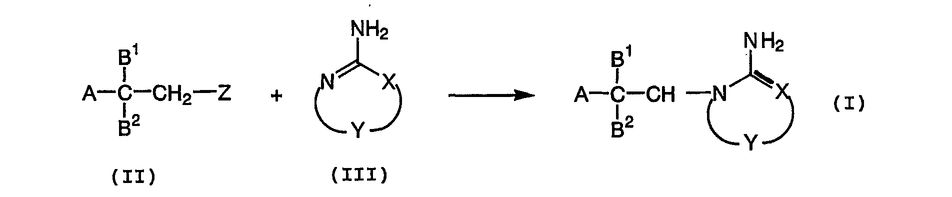 wherein, "Z" is leaving group which accelerates the reaction with nitrogen atoms of heterocyclic ring, such as halogen atom, p-toluenesulfonyloxy, methanesulfonyloxy, trifluoromethanesulfonyloxy, acyloxy, substituted acyloxy groups and so on.

The compound (III) to be used in this reaction can be commercially available or can be easily prepared from known compounds by using common methods.

The reaction of the compound (II) with the compound (III) to obtain the compound (I) can be usually carried out in an appropriate solvent such as alcohol solvent, ketone solvent, nitrile solvent, ester solvent, amide solvent, hydrocarbon solvent and ether solvent or the mixture thereof in the presence of an organic base or an inorganic base if necessary, under the temperature ranging from -20°C to the refluxing temperature of the solvent to be used.

The examples of alcohol solvent include methanol, ethanol, propanol, 2-propanol, 2-methyl-2-propanol and the like. The ketone solvent may include acetone, methyl ethyl ketone and the like. The nitrile solvent may include acetonitrile, propionitrile and so on, and the ester solvent may be ethyl acetate. The examples of amide solvent include N,N-dimethylformamide, N,N-dimethylacetamide, N-methylpyrrolidone, hexamethylphosphoramide and the like. The hydrocarbon solvent may include aromatic hydrocarbon such as benzene, toluene and the like, or aliphatic hydrocarbon such as pentane, hexane and the like. The examples of ether solvent may include diethyl ether, dimethoxyethane, tetrahydrofuran, 1,4-dioxane and the like.

Examples of the organic base to be used in the reaction may include triethylamine, collidine, lutidine, potassium tert-butoxide and the like, and the inorganic base may include potassium carbonate, sodium carbonate, sodium hydrogencarbonate, sodium hydroxide, potassium hydroxide and the like.

### Process 2:

The compound (I) can be obtained by removing the nitro group of the compound (IV) in accordance with the following reaction scheme.

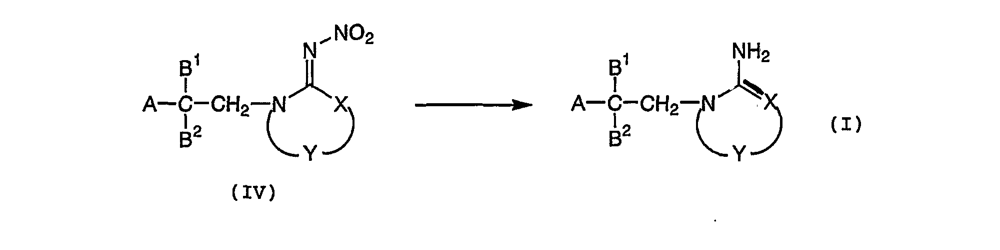

The compound (IV) to be used in this reaction can be prepared in accordance with the known method (Moriya K. et al., *J. Pesticides Sci*., 18, 119-123 (1993)). Removing the nitro group of the compound (IV) can be conducted by using common method such as deprotection of peptides including nitroarginine.

This removing reaction of the nitro group of the compound (IV) can generally be carried out by treating with a reducing reagent in water, or in alcohol solvent, amide solvent, acid solvent alone, or in the mixture solvent thereof, at the temperature ranging from -20°C to 50°C, in the presence of an organic or an inorganic salt having buffer action, if necessary.

The examples of alcohol solvent include methanol, ethanol, propanol, 2-propanol, 2-methyl-2-propanol and the like. The amide solvent include N,N-dimethylformamide, N,N-dimethylacetamide, N-methylpyrrolidone, hexamethylphosphoramide and the like. The acid solvent may include formic acid, acetic acid, propionic acid, trifluoroacetic acid, hydrochloric acid and the like. The examples of the organic or inorganic salt having buffer action may include ammonium acetate, triethylamine, pyridine, phosphate salts and the like. The preferable reducing reagent is titanium(III) chloride.

The compound of formula (I) of the present invention thus obtained can be converted to a pharmaceutically acceptable salt with various kinds of the organic or inorganic acids mentioned above, if necessary. Furthermore, the compound (I) of the present invention can also be purified by the conventional manner, such as recrystallization, column chromatography and the like.

When the compounds of the formula (I) of the present invention exist in the isomer forms, each isomer *per se* is separated from each other by the conventional manner. Therefore, it is understood that each isomers *per se*, as well as the isomeric mixture, shall be included in the compounds of the present invention.

The compounds of formula (I) of the present invention bind selectively to nicotinic acetylcholine receptors in central nervous systems, and activate said receptors as agonists or modulators. Therefore, these compounds are useful as medicaments for preventing or treating various diseases, such as dementia, senile dementia, presenile dementia, Alzheimer's disease, Parkinson's disease, cerebrovascular dementia, AIDS-related dementia, dementia in Down's syndrome, Tourette's syndrome, neurosis during chronic cerebral infarction stage, cerebral dysfunction caused by cerebral injury, anxiety, schizophrenia, depression, Huntington's disease, pain and so on.

The compounds of formula (I) or a pharmaceutically acceptable salt thereof according to the present invention may be administered in the form of oral or parenteral formulations. The formulations for oral administration may include for example, tablets, capsules, granules, fine powders, syrups or the like; the formulations for parenteral administration may include, for example, injectable solutions or suspensions with distilled water for injection or other pharmaceutically acceptable solution, patches for transdermal application, sprays for nasally administration, depositories or the like.

These formulations may be formed by mixing with pharmaceutically acceptable carrier, excipient, sweeter, stabilizer and so on by the conventional procedures known *per se* to those skilled in the art in the field of pharmaceutical formulations.

Examples of pharmaceutically acceptable carrier or excipient include polyvinyl pyrrolidone, gum arabic, gelatin, sorbit, cyclodextrin, magnesium stearate, talc, polyethylene glycol, polyvinyl alcohol, silica, lactose, crystalline cellulose, sugar, starch, calcium phosphate, vegetable oil, carboxymethylcellulose, hydroxypropylcellulose, sodium lauryl sulfate, water, ethanol, glycerol, mannitol, syrup and the like.

The solutions for injection may be isotonic solution containing glucose and the like, and these solutions can be further contain an appropriate solubilizer such as polyethylene glycol or the like, buffer, stabilizer, preservative, antioxidant and so on.

These formulations can be administered to the human being and other mammalian animals, and the preferable administration route may include oral route, transdermic route, nasal route, rectal route, topical route or the like.

The administration dose may vary in a wide range with ages, weights, condition of patients, routes of administration or the like, and a usual recommended daily dose to adult patients for oral administration is within the range of approximately 0.001-1,000 mg/kg per body weight, preferably 0.01-100 mg/kg per body weight, and more preferably 0.1-10 mg/kg per body weight.

In the case of parenteral administration such as intravenous injections, a usual recommended daily dose is within the range of approximately 0.00001-10 mg/kg per body weight, preferably 0.0001-1 mg/kg per body weight, and more preferably 0.001-0.1 mg/kg per body weight, once or in three times per day.

The methods for evaluating the binding capabilities of the compounds at nicotinic acetylcholine receptors are different by subtypes of receptors. The binding capabilities of the compounds at α4β2 nicotinic acetylcholine receptors are examined using rat brain membrane obtained from whole homogenized brain, and determining the inhibiting rate of the compounds against [³H]-cytisine binding to said brain membrane. Furthermore, the binding capabilities of the compounds at α1β1γδ nicotinic acetylcholine receptors are examined using homogenized rat muscle, and determining the inhibiting rate of the compounds against [³H]-α-bungarotoxin binding to said muscle homogenate.

The agonist effect in human α4β2 subtype of nicotinic acetylcholine receptors are examined by using human nicotinic acetylcholine receptors prepared in oocytes of *Xenopus laevis*, which is injected with cRNA from the corresponding cloning cDNA of human α4 and β2 subunits of nicotinic acetylcholine receptors, and to measure the expression of the electric response by adding the test compounds to perfusion solution by means of membrane potential holding method.

### Examples:

The present invention is illustrated in more detail by way of the following examples.

### Reference Example 1: Synthesis by the Process 1

### 3-[2-(6-chloro-3-pyridyl)ethyl]-2-imino-5-methyl-2,3-dihydrothiazole [Compound 6]

A mixture of 440 mg (2 mmol) of 2-amino-5-methyl-2-thiazoline and 228 mg (2 mmol) of 5-(2-bromoethyl)-2-chloropyridine in 5 ml of acetonitrile was heated for 14 hours at 90°C under refluxing. Then, the reaction mixture was cooled to the room temperature, and the solvent was removed under reduced pressure. The resulting residue was treated with methanol and acetone and the precipitates were collected by filtration. The obtained precipitate was purified by aminopropyl-coated silica gel (Chromatorex NH-type; Fuji Silysia Chemical Ltd.) column chromatography (eluent; dichloromethane) to give 310 mg (yield; 61.2%) of 3-[2-(6-chloro-3-pyridyl)ethyl]-2-imino-5-methyl-2,3-dihydrothiazole as colorless oil. This product was dissolved in methanol and to this solution was added 130 mg (1.12 mmol) of fumaric acid, and the mixture was concentrated under reduced pressure. The resulting oily residue was treated with acetone to give crystalline. The crystalline was collected by filtration and dried in vacuum to give 392 mg of fumarate of the title Compound 6.

The following compounds were synthesized in accordance with the same procedures as described in Reference Example 1.
Compound 7: 2-amino-1-[2-(6-chloro-pyridyl)ethyl]imidazole;
Compound 11: 2-amino-1-[2-(6-methyl-3-pyridyl)ethyl]imidazole;
Compound 13: 2-amino-1-[2-(5,6-dichloro-3-pyridyl)ethyl]-imidazole;
Compound 14: 2-amino-1-[2-(3-pyridyl)ethyl]imidazole;
Compound 17: 2-amino-1-[2-(4-chlorophenyl)ethyl]imidazole;
Compound 19: 2-amino-1-[2-(2-pyridyl)ethyl]imidazole;
Compound 20: 2-amino-1-[2-(4-pyridyl)ethyl]imidazole;
Compound 21: 2-amino-1-[2-(6-chloro-3-pyridyl)ethyl]-4,5-dimethylimidazole;
Compound 23: 2-amino-1-[2-(2-chloro-5-thiazolyl)ethyl]imidazole;
Compound 25: 2-amino-1-[2-(5-bromo-3-pyridyl)ethyl]imidazole;
Compound 26: 2-amino-1-[2-(4-hydroxyphenyl)ethyl]imidazole;
Compound 27: 2-amino-1-[2-(5-methyl-3-pyridyl)ethyl]imidazole;
Compound 29: 2-amino-1-[2-(5-pyrimidyl)ethyl]imidazole;
Compound 30: 2-amino-1-[2-(3-pyridazinyl)ethyl]imidazole;
Compound 31: 2-amino-1-[2-(2-pyrazinyl)ethyl]imidazole;
Compound 32: 2-amino-1-{2-[2-(4-hydroxyphenyl)thiophenyl]ethyl}imidazole;
Compound 33: 2-amino-1-{2-[2-(4-methoxyphenyl)thiophenyl]ethyl}imidazole;
Compound 34: 2-amino-1-[2-(4-pyridazinyl)ethyl]imidazole;
Compound 35: 2-amino-1-[2-(4-chloro-5-pyrimidyl)ethyl]imidazole.

### Reference Example 2: Synthesis by the Process 2

### 1-[2-(6-chloro-3-pyridyl)ethyl]-2-imino-1,2,3,4,5,6-hexahydropyrimidine [Compound 12]

To a suspension of 112 mg (0.395 mmol) of 1-[2-(6-chloro-3-pyridyl)ethyl]-2-nitroimino-1,2,3,4,5,6-hexahydropyrimidine in 6 ml of methanol were added 3.64 ml (14.6 mmol) of 4M ammonium acetate and 1.82 ml of 20% titanium(III) chloride, and the mixture was stirred for 1 hour at room temperature under nitrogen atmosphere. Then, the solvent was removed under reduced pressure, and 50% sodium hydroxide aqueous solution was added to the resulting residue under ice-cooling. The insoluble matter was removed off by filtration using Celite, and the filtrate was concentrated under reduced pressure. The resulting residue was purified by aminopropyl-coated silica gel (Chromatorex NH-type; Fuji Silysia Chemical Ltd.) column chromatography (eluent; dichloromethane, then dichloromethane : methanol = 5:1) to give 73 mg (yield; 77.5%) of 1-[2-(6-chloro-3-pyridyl)ethyl]-2-imino-1,2,3,4,5,6-hexahydropyrimidine as colorless oil. This product was dissolved in methanol and to this solution was added 35 mg (0.302 mmol) of fumaric acid, and the mixture was concentrated under reduced pressure. The resulting oily residue was treated with acetone to give crystalline. The crystalline was collected by filtration and dried in vacuum to give 24 mg of fumarate of the title Compound 12.

The physicochemical data of the compound obtained by the above-mentioned examples are summarized in the following Table 1 to Table 7.

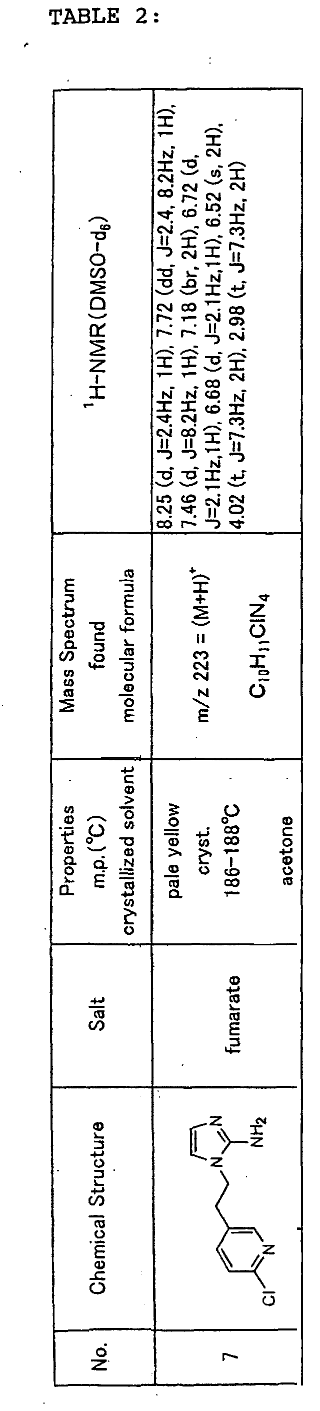

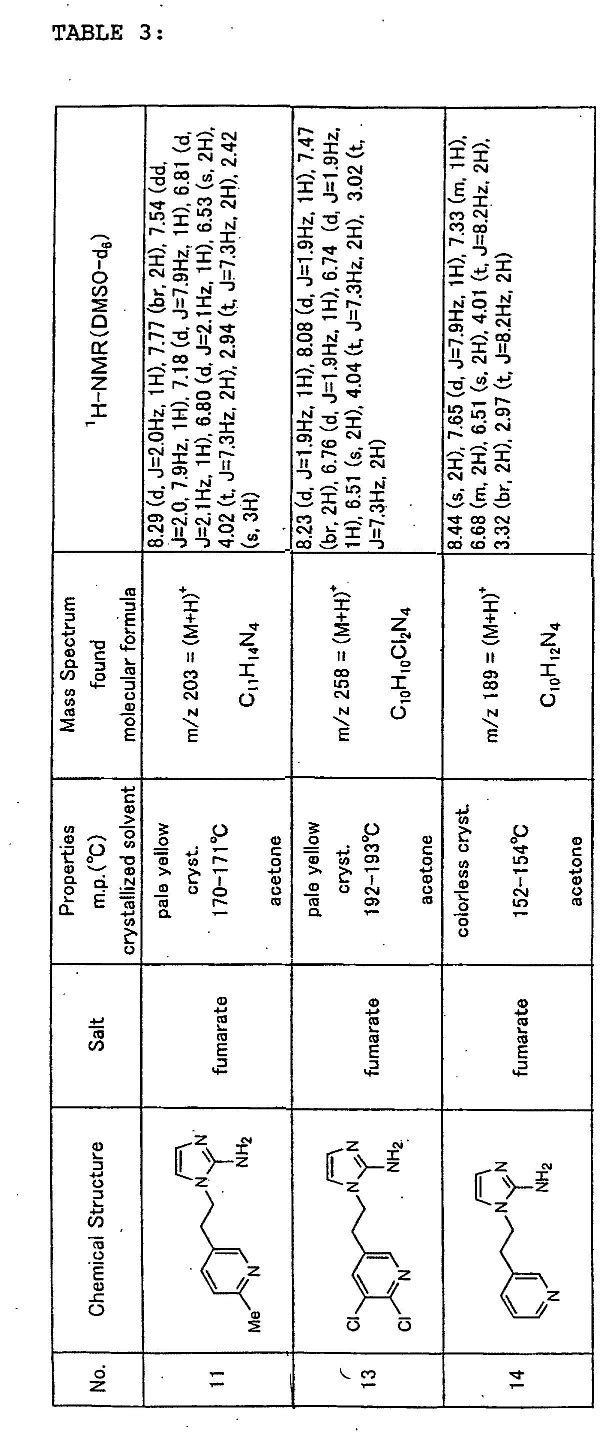

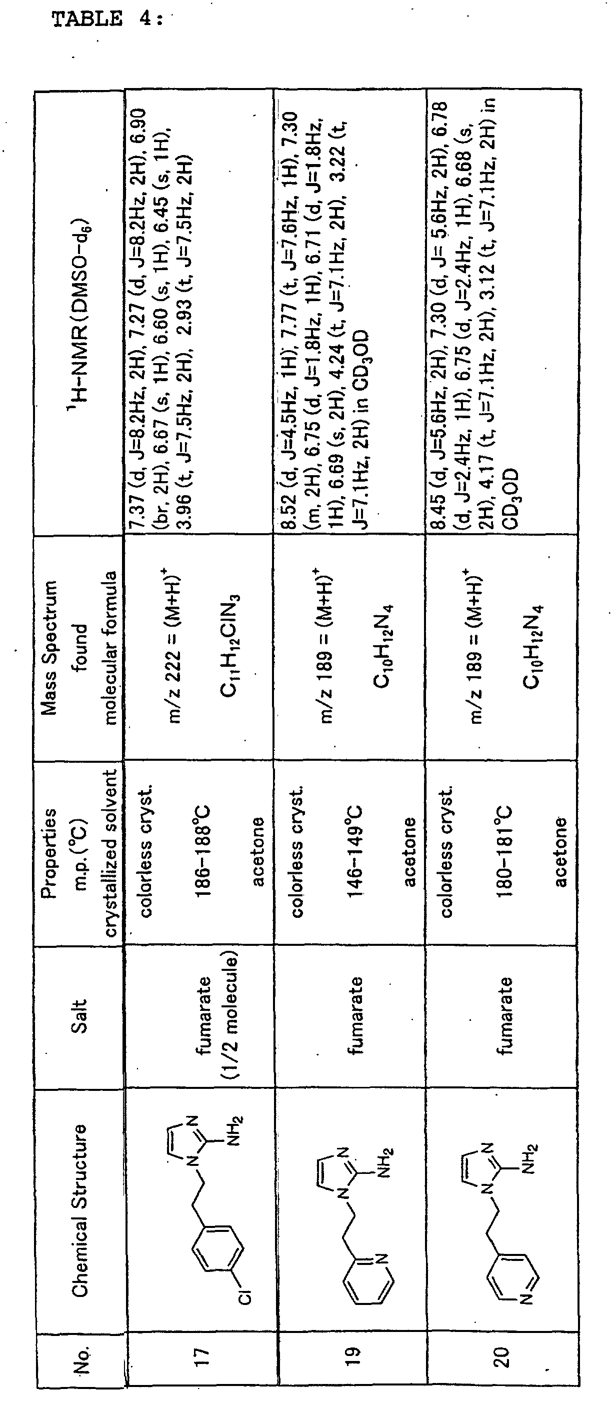

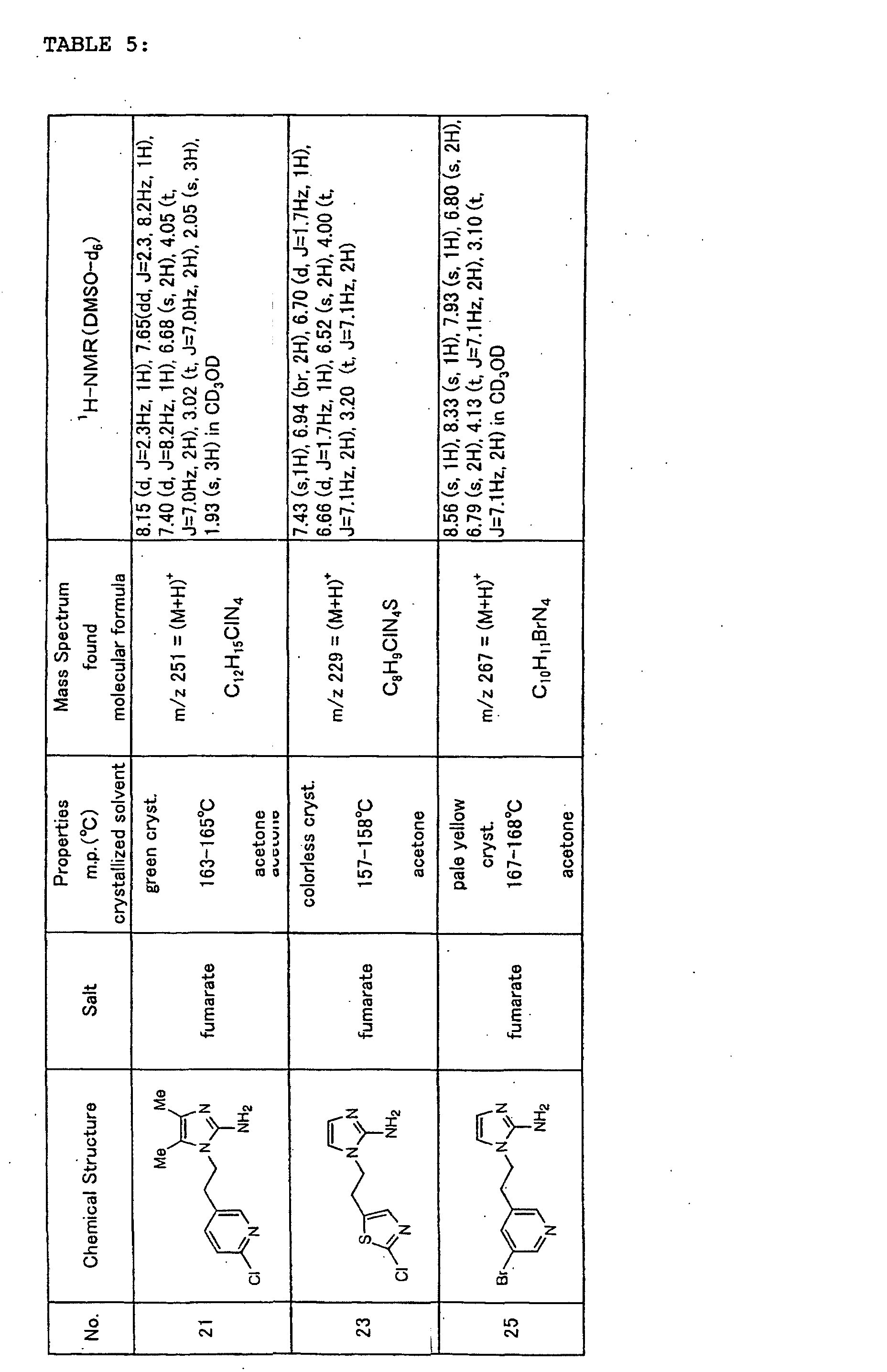

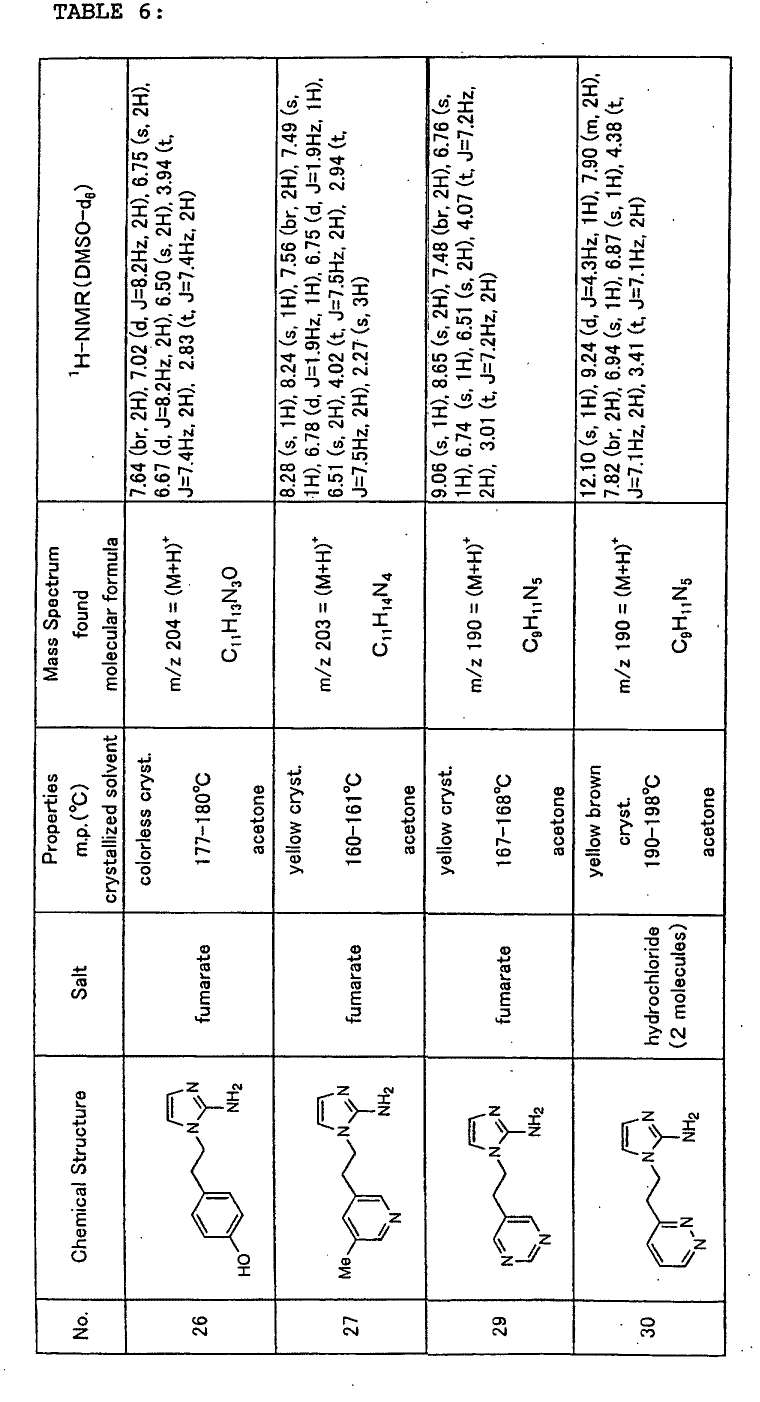

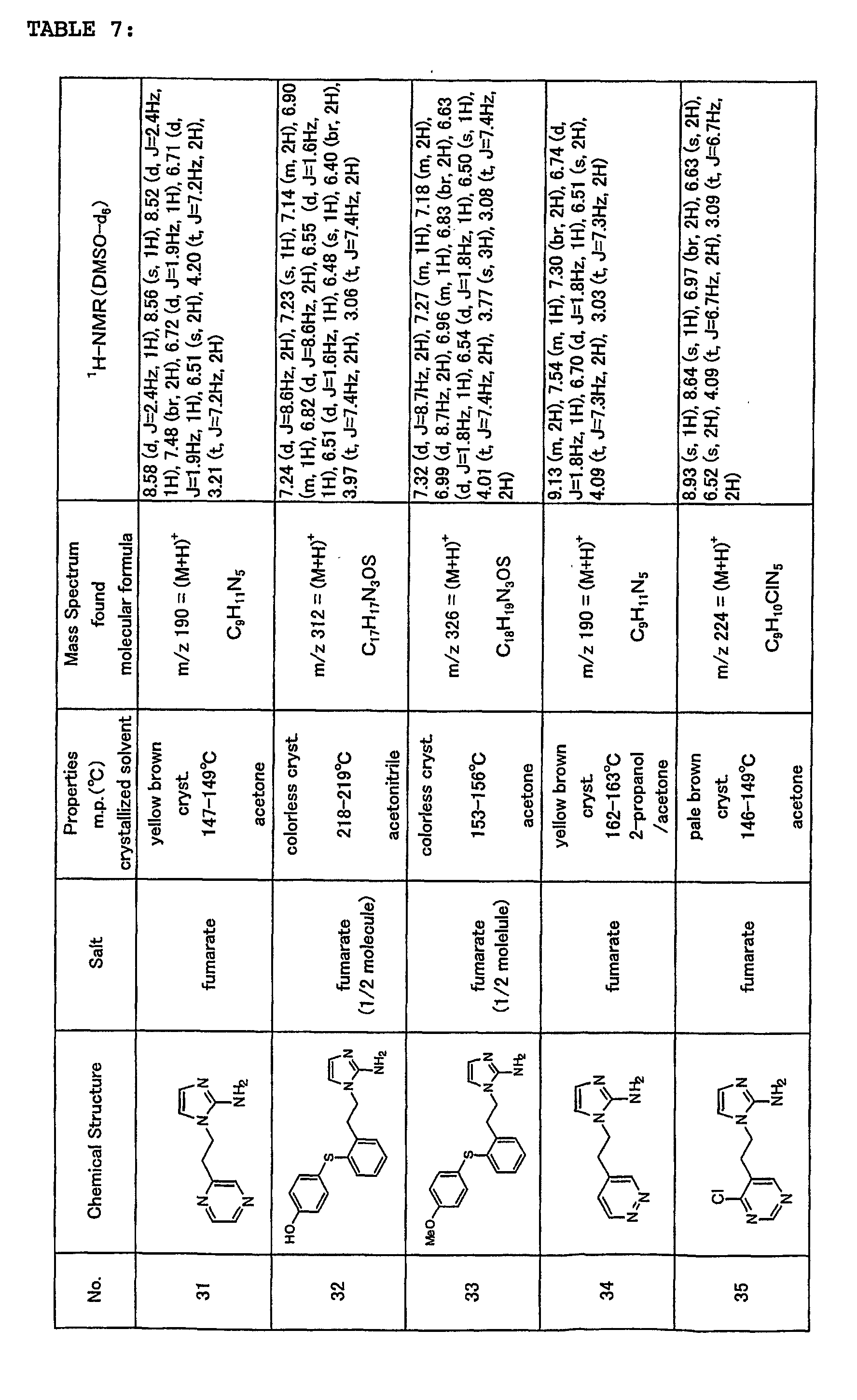

The effect of the compounds (I) of the present invention was evaluated by the following biological experiments.

### Biological Experiment 1:

### Binding assays at α4β2 subtype of nicotinic acetylcholine receptors

The affinity of the compounds of the present invention to α4β2 subtype of nicotinic acetylcholine receptors was performed by the following method, which was modified method described by Pabreza L. A., Dhawan S. & Kellar K. *J., Mol. Pharm*., 39, 9-12 (1990), and by Anderson D. J. & Arneric S. P., *Eur. J. Pharm*., 253, 261-267 (1994).

### (1) Preparation of rat brain membrane containing α4β2 subtype of nicotinic acetylcholine receptors

Fischer-344 strain male rats (body weight: 200-240 g; 9 weeks old) obtained from Charles River Japan were used. Rats were housed in the breeding cage controlled of the room temperature at 23 ± 1°C, and the humidity of 55 ± 5% for 1 to 4 weeks. Rats (3 to 4 rats per a cage) were housed with lights on for 12 hours daily (from 7:00 to 19:00), and allowed free access to food and water.

Preparation of rat brain membrane containing α4β2 subtype of nicotinic acetylcholine receptors was performed as follow. That is, rat brains were isolated just after sacrificed by decapitation, washed with ice-cooled saline solution and then frozen at -80°C with liquid nitrogen and stored till using. After thawing the frozen brain, the brain was homogenized in 10 volumes of ice-cooled buffer solution (50 mM of Tris-HCl, 120 mM of NaCl, 5 mM of KCl, 1 mM of MgCl₂, 2mM of CaCl₂; pH 7.4; 4°C) using homogenizer (HG30, Hitachi Kohki Ltd.) for 30 seconds, and the homogenate were centrifuged under 1,000 x G for 10 minutes at 4°C. The resulting supernatant was separated and the pellet was homogenized again with half volume of aforementioned prior buffer solution and centrifuged under the same conditions. Combined supernatant was further centrifuged under 40,000 x G for 20 minutes at 4°C. The pellet was suspended in buffer solution and used for binding assays at receptors.

### (2) Experiments of α4β2 subtype of nicotinic acetylcholine receptors binding

Suspensions of membrane pellets containing 400-600 µg of protein were added to test tubes containing test compounds and [³H]-cytisine (2 nM) in a final volume of 200 µl and incubated for 75 minutes in ice-cooled bath. The samples were isolated by vacuum filtration onto Whatman GF/B filters, which were prerinsed with 0.5% polyethylenimine just prior to sample filtration, using Brandel multi manifold cell harvester. The filters were rapidly washed with buffer solution (3 x 1 ml). The filters were counted in 3 ml of clearsol I (Nacalai Tesque Inc.). The determination of nonspecific binding was incubated in the presence of 10 µM (-)-nicotine.

The analyses of the experimental results were conducted by using the Accufit Competition Program (Beckman Ltd.).

### Biological Experiment 2:

### Binding assays at α1β1γδ subtype of nicotinic acetylcholine receptors

The affinity of the compounds of the present invention to α1β1γδ subtype of nicotinic acetylcholine receptors was measured by the following method, which was modified method described by Garcha H. S., Thomas P., Spivak C. E., Wonnacott S. & Stolerman I. *P., Psychopharmacology*, 110, 347-354 (1993).

### (1) Preparation of rat skeletal muscles containing α1β1γδ subtype of nicotinic acetylcholine receptors

The substantially same animals described in the Biological Experiment 1 were used.

The isolation of α1β1γδ subtype of nicotinic acetylcholine receptors was performed as follow. That is, rat posterior skeletal muscles were isolated just after sacrificed by decapitation, washed with ice-cooled saline solution and then frozen at -80°C with liquid nitrogen and stored till using. After thawing the frozen muscles, tissue was homogenized (40% w/v) with buffer solution [2.5 mM of sodium phosphate buffer (pH:7.2), 90 mM of NaCl, 2 mM of KCl, 1 mM of EDTA, 2 mM of benzamidine, 0.1 mM of benzethonium chloride, 0.1 mM of PMSF, 0.01% of sodium azide] in Waring blender (Waring blender 34BL97; WARING PRODUCTS DIVISION DYNAMICS CORPORATION OF AMERICA) for 60 seconds. The homogenate were centrifuged under 20,000 x G for 60 minutes at 4°C. The supernatant was separated and the resulting pellet was added to the same buffer (1.5 ml/g wet weight), and homogenized under the same conditions. Triton X100 (2% w/v) was added and the mixture was stirred for 3 hours at 4°C. The centrifugation at 100,000 x G for 60 minutes at 4°C yielded the rat muscle extract as supernatant. This was stored at 4°C for up to 4 weeks, and used for binding assays at receptors.

### (2) Experiments of α1β1γδ subtype of nicotinic acetylcholine receptors binding

Receptors binding experiments were performed as follow. That is, the extract of rat muscle containing 600-900 µg of protein was added to test tubes containing test compounds and incubated for 15 minutes at 37°C. Then, to this mixture was added 1 nM of [³H]-α-bungarotoxin (α-Bgt) and further incubated for 2 hours. The samples were isolated by vacuum filtration onto Whatman GF/B filters, which were prerinsed with 0.5% polyethylenimine just prior to sample filtration, using Brandel multi manifold cell harvester. The filters were rapidly rinsed with washing solution (10 mM of KH₂PO₄, 150 mM of NaCl, pH 7.2, room temperature) (5 x 1 ml). The filters were counted in 3 ml of clearsol I (Nacalai Tesque Inc.). The determination of nonspecific binding was incubated in the presence of 1 µM α-Bgt. The solutions containing α-Bgt (labeled/non-labeled) were prepared by using buffer solution containing 0.25% of BSA. In the receptor binding experiments, said buffer solution was added for adjusting the final concentration of BSA to be 0.05%.

The analyses of the experimental results were conducted by the same way as described in the Biological Experiment 1.

Table 8 shows the results of receptor binding studies of the compounds of the present invention and (-)-nicotine as reference compound.

**TABLE 8:**

| Compound No. | Affinities for receptors Ki | |
|---|---|---|
| | α4β2 ^{*1} | α1β1γδ ^{**2} |
| 7 | 4.9 nM | 184 µM |
| 11 | 21 nM | 1150 µM |
| 13 | 3.1 nM | 40 µM |
| 14 | 3.5 nM | 223 µM |
| 17 | (97%, 78%) | (83%, 67%) |
| 19 | (103%, 106%) | (106%, 86%) |
| 20 | (97%, 95%) | (109%, 90%) |
| 21 | 97 nM | 175 µM |
| 23 | 8.5 nM | 44 µM |
| 25 | 2 nM | 36 µM |
| 26 | (93%, 60%) | (81%, 50%) |
| 27 | 21 nM | 339 µM |
| 29 | 6.0 nM | 447 µM |
| 30 | 40 nM | 80 µM |
| 31 | (75%, 22%) | (110%, 53%) |
| 32 | (102%, 73%) | (107%, 81%) |
| 33 | (111%, 92%) | (119%, 85%) |
| 34 | 68 nM | 1266 µM |
| 35 | (61%, 17%) | (87%, 49%) |
| Nicotine | 1.6 nM | 182 µM |

| | | |
|---|---|---|
| *¹: Values indicated in a parenthesis show control % of [³H]-cytisine binding at 1 µM and 10 µM of test compounds, respectively. | | |
| **² : Values indicated in a parenthesis show control % of [³H]-α-Bgt binding at 100 µM and 1,000 µM of test compounds. n.d.: not determined. | | |

### Biological Experiment 3:

### Agonist activities at human α4β2 subtype of nicotinic acetylcholine receptors

The agonist activities of the compounds of the present invention at human α4β2 subtype of nicotinic acetylcholine receptors was evaluated by the following method, which was modified method described by Papke R. L., Thinschmidt J. S., Moulton B. A., Meyer E. M. & Poirier A., *Br. J. Pharmacol*., 120, 429-438 (1997).

### (1) Preparation of cRNA of human α4β2 subtype of nicotinic acetylcholine receptors

The cloning of human nicotinic acetylcholine receptor (hnACh-R) α4 cDNA and hnAC-R β2 cDNA were performed, in accordance with the conventional manners, by synthesizing the each DNA primers corresponding to the sequences of hnACh-R α4 cDNA and hnACh-R β2 cDNA [Monteggia L. M. et al., *Gene*, 155, 189-193 (1995); and Anand R., & Lindstrom J., *Nucl. Acids Res.,* 18, 4272 (1990)], and obtained hnACh-R α4 cDNA and hnACh-R β2 cDNA by polymerase chain reaction (PCR), respectively. The obtained hnACh-R α4 cDNA and hnACh-R β2 cDNA were inserted to the cRNA expression vector (pSP64 polyA) having SP6 RNA promoter to construct hnACh-R α4/pSP64 polyA and hnACh-R β2/pSP64 polyA, respectively. After cutting from expression vector by restriction enzyme (EcoRI), transcription was performed by affecting SP6 RNA polymerase in the presence of cap analogues to obtain hnACh-R α4 cRNA and hnACh-R β2 cRNA, respectively.

### (2) Expression of human α4β2 subtype nicotinic acetylcholine receptors in Xenopus oocytes

Oocytes were purchased from Kitanihonseibutsukyohzai Co., Ltd., which were already enucleated from *Xenopus laevis*, and used in this experiment.

The oocytes were treated with collagenase (Sigma type I; 1 mg/ml) in calcium-free modified Birth's solution (88 mM of NaCl, 1 mM of KCl, 2.4 mM of NaHCO₃, 0.82 mM of MgSO₄, 15 mM of HEPES, pH 7.6) under gently stirring at room temperature for 90 minutes, and washed out the enzyme from the tissue. Then, oocytes were separated from ovarian follicle by tweezers, and isolated oocytes were placed in antibiotics containing modified Birth's solution (88 mM of NaCl, 1 mM of KCl, 2.4 mM of NaHCO₃, 0.82 mM of MgSO₄, 15 mM of HEPES, pH 7.6, and 0.1 v/v% of mixture solution containing of penicillin and streptomycin for incubation; Sigma Co.). Thus treated oocytes were injected with 50 nl of adjusted cRNAs (1.0 mg/ml), that is, each 50 ng of hnACh-R α4 cRNA and hnACh-R β2 cRNA per 1 oocyte by using automatic injector (NANOJECT; DRUMMOND SCIENTIFIC CO.), and further incubated for 4-14 days at 19°C. In oocytes, heterogeneous quintuple [(α4)₂(β2)₃] was composed by translation of injected cRNAs, and ion channel receptors were constructed on cell membrane.

### (3) Agonist activities at human α4β2 subtype of nicotinic acetylcholine receptors

The recordings of responses at human α4β2 subtype of nicotinic acetylcholine receptors by means of membrane potential holding method were performed as follow. That is, oocytes were placed in recording chamber with a total volume of 50 µl and were perfused with Ringer's solution (115 mM of NaCl, 2.5 mM of KCl, 1.8 mM of CaCl₂, 10 mM of HEPES, pH 7.3) containing atropine (1 µM) under flow rate of 1 ml/min. The membrane electric potentials were held at -50 mV by mean of the two electric membranes potential holding method (CEZ-1250; Nihon Kohden Co.). Test compounds were added to the perfusion solution, and recorded the peak strength of induced inward current. In order to normalize the responses of test compounds, the responses with acetylcholine (Ach) were recorded before and after application of the test compounds. Generally in the oocytes just after isolated, the response of intrinsic muscarinic acetylcholine receptors, which is inward electric current caused by activation of calcium dependence chloride ion channels with increase of the intracellular calcium concentration by stimulation of receptors, is observed. However, the complete disappearances of these responses were confirmed when treated with collagenase or added 1 µM of atropine. Furthermore, the oocytes without injection of cRNAs showed no responses by Ach after treatment with collagenase. Therefore, the responses observed in oocytes with injection of hnACh-R α4 cRNA and hnACh-R β2 cRNA, i.e., the inward current induced by the intracellular influx of sodium ion according to the stimulation of receptors, would be the freshly observed responses of human α4β2 subtype nicotinic acetylcholine receptors.

Table 9 shows the results of the agonist activity test of the compounds in the present invention and (-)-nicotine as reference compound.

**TABLE 9:**

| Compound No. | Agonist activity (ED50)^{*1} |
|---|---|
| 7 | 2.5 µM |
| 11 | 7.5 µM |
| 13 | 2.4 µM |
| 14 | 2.0 µM |
| 23 | 13 µM |
| 25 | 3.0 µM |
| 27 | 6.2 µM |
| 29 | 3.7 µM |
| 30 | 46 µM |
| 31 | (15%) |
| 34 | 170 µM |
| nicotine | 11.4 µM |

| | |
|---|---|
| *¹: These date are shown in control % by response at 100 µM of the test compounds, in comparison with the response at 10 µM of acetylcholine (100%). Values indicated in a parenthesis show control % by response at 100 µM of the test compounds. | |

The following are Formulation Examples of the compounds (I) or pharmaceutically acceptable salt thereof according to the present invention

### Formulation Example 1 (Tablets):

| | |
|---|---|
| Compound 7 (Fumarate) | 25 g |
| Lactose | 130 g |
| Crystalline cellulose | 20 g |
| Corn starch | 20 g |
| 3% aqueous solution of hydroxypropylmethylcellulose | 100 ml |
| Magnesium stearate | 2 g |

Fumarate of Compound 7, lactose, crystalline cellulose and corn starch were screened through a 60-mesh sieve, homogenized and charged into a kneader. A 3% aqueous solution of hydroxypropylmethylcellulose was added to the homogeneous mixture and the mixture was further kneaded. The product was granulated by a 16-mesh sieve, dried in air at 50°C, and again granulated by a 16-mesh sieve. Magnesium stearate was added to the granule and mixed again. The mixture was tabletted to produce tablets weighing 200 mg each and having an 8 mm diameter.

### Formulation Example 2 (Capsules):

| | |
|---|---|
| Compound 13 (Fumarate) | 25.0 g |
| Lactose | 125.0 g |
| Corn starch | 48.5 g |
| Magnesium stearate | 1.5 g |

The above components were finely pulverized and thoroughly mixed to produce a homogeneous mixture. The mixture was filled in gelatin capsules, 200 mg per capsule, to obtain capsules.

### Formulation Example 3 (Injection):

The fumarate of Compound 29 was filled in an amount of 250 mg in a vial and mixed in situ with approximately 4-5 ml of injectable distilled water to make an injectable solution.

### INDUSTRIAL APPLICABILITY

As described above, the compounds of the present invention possess high affinity for α4β2 nicotinic acetylcholine receptor of central nervous systems and activate said α4β2 nicotinic acetylcholine receptor as agonists or modulators. Therefore, the compounds of the present invention are useful for preventing or treating various kinds of diseases, which may be prevented or cured by activating nicotinic acetylcholine receptors.

Especially, the activators for α4β2 nicotinic acetylcholine receptors of the present invention are useful for preventing or treating various diseases such as dementia, senile dementia, presenile dementia, Alzheimer's disease, Parkinson's disease, cerebrovascular dementia, AIDS-related dementia, dementia in Down's syndrome, Tourette's syndrome, neurosis during chronic cerebral infarction stage, cerebral dysfunction caused by cerebral injury, anxiety, schizophrenia, depression, Huntington's disease, pain and so on.

## Claims

1. Heterocyclic compounds represented by the following formula (I): 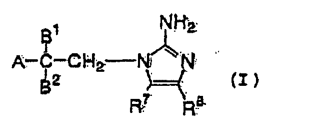 wherein:
A is alkyl group selected from methyl, ethyl, propyl, isopropyl, butyl, sec-butyl and tert-butyl; an aryl group which may be substituted by a substituent selected from halogen atom, C₁-C₄ alkyl group, hydroxyl group, alkylthio group and arylthio group; or 5- or 6-membered heterocyclic group or condensed heterocyclic group thereof containing the same or different one to three heteroatom(s) of sulfur, nitrogen or oxygen atom(s), which may be substituted by C₁-C₄ alkyl group or halogen atom;
B¹ and B² are hydrogen atom; alkyl group; hydroxyl group; or combined together to represent carbonyl group;
R⁷ and R⁸ are hydrogen atom; halogen atom; alkyl group selected from methyl, ethyl, propyl, isopropyl, butyl, sec-butyl and tert-butyl; aryl group which is either non-substituted phenyl group or phenyl group which is substituted by halogen atom or C₁-C₄ alkyl group; or 5- or 6-membered heterocyclic group containing the same or different one to three hetero atom(s) of sulfur, nitrogen or oxygen atom(s), which may be substituted by C₁-C₄ alkyl group or halogen atom;
or pharmaceutically acceptable salts thereof.

2. The following compounds represented by the formula (I) of claim 1:
2-amino-1-[2-(6-chloro-3-pyridyl)ethyl]imidazole;
2-amino-1-[2-(6-methyl-3-pyridyl)ethyl]imidazole;
2-amino-1-[2-(5,6-dichloro-3-pyridyl)ethyl]imidazole;
2-amino-1-[2-(3-pyridyl)ethyl]imidazole;
2-amino-1-[2-(4-chlorophenyl)ethyl]imidazole;
2-amino-1-[2-(2-pyridyl)ethyl]imidazole;
2-amino-1-[2-(4-pyridyl)ethyl]imidazole;
2-amino-1-[2-(6-chloro-3-pyridyl)ethyl]-4,5-dimethylimidazole;
2-amiao-1-[2-(2-chloro-5-thiazolyl)ethyl]imidazole;
2-amino-1-[2-(5-bromo-3-pyridyl)ethyl]imidazole;
2-amino-1-[2-(4-hydroxyphenyl)ethyl]imidazole;
2-amino-1-[2-(5-methyl-3-pyridyl)ethyl]imidazole;
2-amino-1-[2-(5-pyrimidyl)ethyl]imidazole;
2-amino-1-[2-(3-pyridazinyl)ethyl]imidazole;
2-amino-1-[2-(2-pyrazinyl)ethyl]imidazole;
2-amino-1-{2-[2-(4-hydroxyphenyl)thiophenyl]ethyl}imidazole;
2-amino-1-{2-[2-(4-methoxyphenyl)thiophenyl]ethyl}imidazole;
2-amino-1-[2-(4-pyridazinyl)ethyl]imidazole;
2-amino-1-[2-(4-chloro-5-pyrimidyl)ethyl]imidazole;
and pharmaceutically acceptable salt thereof.

3. Activators for α4β2 nicotinic acetylcholine receptors containing the compound or pharmaceutically acceptable salt thereof claimed in claim 1 or 2, as active ingredient.

4. The activators for α4β2 nicotinic acetylcholine receptors according to claim 3, wherein said activators are agonists or modulators at α4β2 nicotinic acetylcholine receptors.

5. Use of a compound according to claim 1 or 2 for the preparation of a medicament for preventing or treating cerebral circulation diseases.

6. Use of a compound according to claim 1 or 2 for the preparation of a medicament for preventing or treating neurodegenerative disease, dementia, motor ataxia, and neuropathy and mental disease.

7. Use according to claim 6, wherein said neurodegenerative disease is Alzheimer's disease or Parkinson's disease, said dementia is cerebrovascular dementia, said motor ataxia is Tourette's syndrome, and said neuropathy and mental disease is neurosis during chronic cerebral infarction stage, anxiety or schizophrenia.

8. Use of a compound according to claim 1 or 2 for the preparation of a medicament for improving the cerebral metabolism, neurotransmission functional disorder and memory disorder, for protecting brain, or having analgesic effect.

9. Use of a compound according to claim 1 or 2 for the preparation of a medicament for preventing or treating inflammatory intestinal diseases.

10. A pharmaceutical composition comprising a compound according to claim 1 or 2.

## Patentansprüche

1. Heterocyclische Verbindungen der folgenden Formel (I): 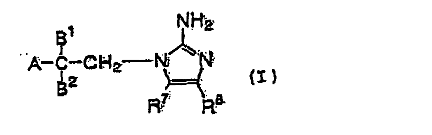 wobei:
A ein aus einer Methyl-, Ethyl-, Propyl-, Isopropyl-, Butyl-, sek.-Butyl- und tert.-Butylgruppe ausgewählter Alkylrest; ein Arylrest, der mit einem aus einem Halogenatom, C₁-C₄-Alkylrest, Hydroxylrest, Alkylthiorest und Arylthiorest ausgewählten Substituenten substituiert sein kann; oder ein 5- oder 6-gliedriger heterocyclischer Rest oder kondensierter heterocyclischer Rest davon ist, der das gleiche oder verschiedene ein bis drei Heteroatom(e) aus Schwefel-, Stickstoff- oder Sauerstoffatom(en) enthält und mit einem C₁-C₄-Alkylrest oder Halogenatom substituiert sein kann;
B¹ und B² ein Wasserstoffatom, einen Alkylrest oder eine Hydroxylgruppe bedeuten oder miteinander verbunden sind, so dass sie eine Carbonylgruppe darstellen;
R⁷ und R⁸ ein Wasserstoffatom, ein Halogenatom, einen aus Methyl-, Ethyl-, Propyl-, Isopropyl-, Butyl-, sek.-Butyl- und tert.-Butylgruppe ausgewählten Alkylrest, einen Arylrest, der entweder ein unsubstituierter Phenylrest oder ein Phenylrest ist, der mit einem Halogenatom oder einem C₁-C₄-Alkylrest substituiert ist; oder einen 5- oder 6-gliedrigen heterocyclischen Rest bedeuten, der das gleiche oder verschiedene ein bis drei Heteroatom(e) aus Schwefel-, Stickstoff- oder Sauerstoffatom(en) enthält und mit einem C₁-C₄-Alkylrest oder Halogenatom substitutiert sein kann;
oder pharmazeutisch verträgliche Salze davon.

2. Die folgenden Verbindungen der Formel (I) nach Anspruch 1:
2-Amino-1-[2-(6-chlor-3-pyridyl)ethyl]imidazol;
2-Amino-1-[2-(6-methyl-3-pyridyl)ethyl]imidazol;
2-Amino-1-[2-(5,6-dichlor-3-pyridyl)ethyl]imidazol;
2-Amino-1-[2-(3-pyridyl)ethyl]imidazol;
2-Amino-1-[2-(4-chlorphenyl)ethyl]imidazol;
2-Amino-1-[2-(2-pyridyl)ethyl]imidazol;
2-Amino-1-[2-(4-pyridyl)ethyl]imidazol;
2-Amino-1-[2-(6-chlor-3-pyridyl)ethyl]-4,5-dimethylimidazol;
2-Amino-1-[2-(2-chlor-5-thiazolyl)ethyl]imidazol;
2-Amino-1-[2-(5-brom-3-pyridyl)ethyl]imidazol;
2-Amino-1-[2-(4-hydroxyphenyl)ethyl]imidazol;
2-Amino-1-[2-(5-methyl-3-pyridyl)ethyl]imidazol;
2-Amino-1-[2-(5-pyrimidyl)ethyl]imidazol;
2-Amino-1-[2-(3-pyridazinyl)ethyl]imidazol;
2-Amino-1-[2-(2-pyrazinyl)ethyl]imidazol;
2-Amino-1-{2-[2-(4-hydroxyphenyl)thiophenyl]ethyl}imidazol;
2-Amino-1-{2-[2-(4-methoxyphenyl)thiophenyl]ethyl}imidazol;
2-Amino-1-[2-(4-pyridazinyl)ethyl]imidazol;
2-Amino-1-[2-(4-chlor-5-pyrimidyl)ethyl]imidazol;
und pharmazeutisch verträgliches Salz davon.

3. Aktivatoren für α4β2-Nicotinacetylcholin-Rezeptoren, die die Verbindung oder das pharmazeutisch verträgliche Salz davon nach Anspruch 1 oder 2 als Wirkstoff enthalten.

4. Aktivatoren für α4β2-Nicotinacetylcholin-Rezeptoren nach Anspruch 3, wobei die Aktivatoren Agonisten oder Modulatoren an α4β2-Nicotinacetylcholin-Rezeptoren sind.

5. Verwendung einer Verbindung nach Anspruch 1 oder 2 für die Herstellung eines Medikaments zur Verhinderung oder Behandlung zerebraler Durchblutungsstörungen.

6. Verwendung einer Verbindung nach Anspruch 1 oder 2 für die Herstellung eines Medikaments zur Verhinderung oder Behandlung neurodegenerativer Erkrankung, Dementia, motorischer Ataxie und Neuropathie sowie Geisteskrankheit.

7. Verwendung nach Anspruch 6, wobei die neurodegenerative Erkrankung Alzheimer-Krankheit oder Parkinson-Krankheit ist, die Dementia zerebrovaskuläre Dementia ist, die motorische Ataxie Tourette-Syndrom ist und die Neuropathie und Geisteskrankheit Neurose im chronischen Stadium eines zerebralen Infarkts, Angst oder Schizophrenie sind.

8. Verwendung einer Verbindung nach Anspruch 1 oder 2 für die Herstellung eines Medikaments zur Verbesserung des Himstoffwechsels, der Neurotransmissionsfunktionsstörung und Gedächtnissstörung, zum Schutz des Gehirns oder mit analgetischer Wirkung.

9. Verwendung einer Verbindung nach Anspruch 1 oder 2 zur Herstellung eines Medikaments zur Verhinderung oder Behandlung entzündlicher Darmerkrankungen.

10. Arzneimittel umfassend eine Verbindung nach Anspruch 1 oder 2.

## Revendications

1. Composés hétérocycliques représentés par la formule (I) suivante : 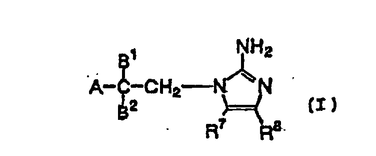 dans laquelle :
A est un groupe alkyle choisi parmi méthyle, éthyle, propyle, isopropyle, butyle, sec-butyle et tert-butyle ; un groupe aryle qui peut être substitué par un substituant choisi parmi un atome d'halogène, un groupe alkyle en C₁-C₄, un groupe hydroxyle, un groupe alkylthio et un groupe arylthio ; ou un groupe hétérocyclique à 5 ou 6 membres ou un groupe hétérocyclique condensé de celui-ci contenant un à trois hétéroatome(s), identique(s) ou différent(s), de soufre, azote ou oxygène, qui peut être substitué par un groupe alkyle en C₁-C₄ ou un atome d'halogène ;
B¹ et B² sont un atome d'hydrogène, un groupe alkyle, un groupe hydroxyle, ou combinés ensemble représentent un groupe carbonyle ;
R⁷ et R⁸ sont un atome d'hydrogène, un atome d'hydrogène, un groupe alkyle choisi parmi méthyle, éthyle, propyle, isopropyle, butyle, sec-butyle et tert-butyle ; un groupe aryle qui est soit un groupe phényle non substitué soit un groupe phényle qui est substitué par un atome d'halogène ou par un groupe alkyle en C₁-C₄ ; ou un groupe hétérocyclique à 5 ou 6 membres contenant un à trois hétéroatome(s), identique(s) ou différent(s), de soufre, d'azote ou d'oxygène, qui peut être substitué par un groupe alkyle en C₁-C₄ ou par un atome d'halogène ;
ou des sels acceptables sur le plan pharmaceutique de ceux-ci.

2. Les composés suivants représentés par la formule (I) de la revendication 1 :
2-amino-1-[2-(6-chloro-3-pyridyl)éthyl]imidazole ;
2-amino-1-[2-(6-méthyl-3-pyridyl)éthyl]imidazole ;
2-amino-1-[2-(5,6-dichloro-3-pyridyl)éthyl]imidazole ;
2-amino-1-[2-(3-pyridyl)éthyl]imidazole ;
2-amino-1-[2-(4-chlorophényl)éthyl]imidazole ;
2-amino-1-[2-(2-pyridyl)éthyl]imidazole ;
2-amino-1-[2-(4-pyridyl)éthyl]imidazole ;
2-amino-1-[2-(6-chloro-3-pyridyl)éthyl]-4,5-diméthylimidazole ;
2-amino-1-[2-(2-chloro-5-thiazolyl)éthyl]imidazole ;
2-amino-1-[2-(5-bromo-3-pyridyl)éthyl]imidazole ;
2-amino-1-[2-(4-hydroxyphényl)éthyl]imidazole ;
2-amino-1-[2-(5-méthyl-3-pyridyl)éthyl]imidazole ;
2-amino-1-[2-(5-pyrimidyl)éthyl]imidazole ;
2-amino-1-[2-(3-pyridazinyl)éthyl]imidazole ;
2-amino-1-[2-(2-pyrazinyl)éthyl]imidazole ;
2-amino-1-{2-[2-(4-hydroxyphényl)thiophényl]éthyl}imidazole ;
2-amino-1-{2-[2-(4-méthoxyphényl)thiophényl]éthyl}imidazole ;
2-amino-1-[2-(4-pyridazinyl)éthyl]imidazole ;
2-amino-1-[2-(4-chloro-5-pyrimidyl)éthyl]imidazole ;
et un sel acceptable sur le plan pharmaceutique de ceux-ci.

3. Activateurs des récepteurs nicotiniques à l'acétylcholine α4β2 contenant le composé ou sel acceptable sur le plan pharmaceutique de celui-ci revendiqué dans la revendication 1 ou 2, en tant qu'agent actif.

4. Activateurs des récepteurs nicotiniques à l'acétylcholine α4β2 selon la revendication 3, lesdits activateurs étant des agonistes ou des modulateurs des récepteurs nicotiniques à l'acétylcholine α4β2.

5. Utilisation d'un composé selon la revendication 1 ou 2 pour la préparation d'un médicament pour prévenir ou traiter les maladies de la circulation cérébrale.

6. Utilisation d'un composé selon la revendication 1 ou 2 pour la préparation d'un médicament pour prévenir ou traiter une maladie neurodégénérative, la démence, l'ataxie moteur, et les maladies neuropathiques et mentales.

7. Utilisation selon la revendication 6, dans laquelle ladite maladie neurodégénérative est la maladie d'Alzheimer ou la maladie de Parkinson, ladite démence est la démence cérébro-vasculaire, ladite ataxie moteur est le syndrome de Gilles de la Tourette et ladite maladie neuropathique et mentale est la névrose au stade chronique de l'infarctus cérébral, l'anxiété ou la schizophrénie.

8. Utilisation d'un composé selon la revendication 1 ou 2 pour la préparation d'un médicament pour améliorer le métabolisme cérébral, les désordres fonctionnels de la neurotransmission et les désordres de la mémoire, pour protéger le cerveau ou pour un effet analgésique.

9. Utilisation d'un composé selon la revendication 1 ou 2 pour la préparation d'un médicament pour prévenir ou traiter les maladies inflammatoires intestinales.

10. Composition pharmaceutique comprenant un composé selon la revendication 1 ou 2.
